# EUROPEAN PATENT APPLICATION

(11) **EP 3 506 721 A1**
(43) Date of publication of application: **03.07.2019**
(21) Application number: 18215813.9
(22) Date of filing: 22.12.2018
(51) Int. Cl.: H05B 6/44, A24F 47/00

(54) **ELECTROMAGNETIC HEATING DEVICE AND SMOKING SET HAVING SAME**

(30) Priority: 22.12.2017 CN 201721823986 U; 22.12.2017 CN 201721838946 U
(71) Applicant: Shenzhen First Union Technology Co., Ltd., 518103 Shenzhen, Guangdong (CN)
(72) Inventor: WU, Zexin, Shenzhen, Guangdong 518103 (CN); XU, Zhongli, Shenzhen, Guangdong 518103 (CN); LI, Yonghai, Shenzhen, Guangdong 518103 (CN)
(74) Representative: Proi World Intellectual Property GmbH

(57) **Abstract**

A heating device and a low-temperature baked smoking set are disclosed. The heating device includes a heating compartment and an electromagnetic inductive coil that is wound around a periphery of the heating compartment. The electromagnetic inductive coil at least includes a first inductive coil and a second inductive coil; the first inductive coil and the second inductive coil are wound around two parts of the periphery of the heating compartment, extending in an axial direction of the heating compartment.

Turns of the first inductive coil is more than turns of the second inductive coil such that heating speeds of two parts of the periphery of the heating compartment are different.

## Description

### TECHNICAL FIELD

The present disclosure relates to the field of smoking sets, and particularly to an electromagnetic heating device and a smoking set having same.

### BACKGROUND ART

Currently, with the increasing number of the smokers, tobacco cigarettes are widely appeared in our daily life, it is difficult to avoid passive smoking. The traditional tobacco cigarettes needs open fire to be lit to produce smoking smog, as used herein, the tobacco cigarette emits substantive amount of toxic materials to human beings. A majority of smokers know the detriment of smoking but can't self-control, so quitting smoking has been always up in the air.

In the recent years, a kind of low-temperature baked smoking set is appeared, with low-temperature baking to produce the smoking smog, greatly reducing generation of toxic materials. The low-temperature baked smoking set in the market generally includes a heating device, into which the tobacco cigarette is inserted, by relying on the heater heating the tobacco cigarette, the smoking set generates smoking smog.

Thus, the related smoking sets to the inventors have following disadvantages: the heating device in the related smoking set may evenly heat a disposable tobacco cigarette such that smelling and flavors in latter stage get diminished, therefore, during smoking one tobacco cigarette, the smelling and flavors are inconsistent in former and latter stages to influence the user experience of smoking.

### SUMMARY

In view of the drawbacks in the prior art, the present disclosure relates to an electromagnetic heating device and a smoking set having the same, with simple structures and available for sectional heating such that smelling and flavors will be consistent during tobacco cigarettes are drawn.

In order to solve the above technical problem, the present disclosure provides a heating device according to independent claim 1 whereas various embodiments of the heating device and improvements thereto are recited in the dependent claims. The heating device includes a heating compartment; an electromagnetic inductive coil wound around a periphery of the heating compartment; the electromagnetic inductive coil at least comprises a first inductive coil and a second inductive coil; the first inductive coil and the second inductive coil are wound around two parts of the periphery of the heating compartment, the first inductive coil and the second inductive coil extending in an axial direction of the heating compartment; turns of the first inductive coil is more than turns of the second inductive coil such that heating speeds of two parts of the periphery of the heating compartment are different.

As used herein, the electromagnetic inductive coil further includes a connective coil, respectively connected with the first inductive coil and the second inductive coil.

As used herein, the first inductive coil and the second inductive coil are made by one same electricity conductive wire.

As used herein, the heating device further includes a paramagnet, disposed outside the heating compartment and the electromagnetic inductive coil, extending along an axial direction of the spiral electromagnetic inductive coil; the paramagnet is configured for changing a direction of magnetic field lines of the electromagnetic inductive coil.

As used herein, the heating compartment further includes a metallic heating tube and a wire-wound supporter; the heating tube is configured for receiving aerosol-formed materials; the wire-wound supporter is sleeved outside the heating tube, the electromagnetic inductive coil is wound around the supporter; the paramagnet is disposed outside the supporter.

As used herein, the heating compartment includes a wire-wound supporter, a metallic heating tube and a base, the heating tube is configured for receiving aerosol-formed materials; the supporter is sleeved outside the heating tube and the base, the electromagnetic inductive coil is wound around the supporter; the paramagnet is disposed outside the supporter.

As used herein, the supporter includes a slot, the slot includes a first slot and a second slot, part of the first inductive coil is inserted into the first slot and part of the second inductive coil is inserted into the second slot.

As used herein, the paramagnet includes a pressing part, a first fixing part and a second fixing part; the first fixing part and the second fixing part are respectively disposed at two ends of the pressing part; the first fixing part is fixed with one end of the heating compartment, the second fixing part is fixed with the other end of the heating compartment; the pressing part and the heating compartment clamp the electromagnetic inductive coil therebetween.

As used herein, a number of the paramagnets is multiple, multiple paramagnets are evenly distributed around the heating compartment.

As used herein, the paramagnet is hollow and a tubular element with an open end, the paramagnet is sleeved on the heating compartment, the paramagnet and the heating compartment clamp the electromagnetic inductive coil therebetween.

As used herein, the paramagnet is made of at least one or more selected from a group of manganese ferrite, zinc ferrite, nickel-zinc ferrite and manganese-magnesium-zinc ferrite.

To solve the above problem, the present disclosure further provides a low-temperature baked smoking set having the heating device as described supra.

As used herein, the smoking set further includes a power supply module, the power supply module is coupled with the electromagnetic inductive coil in the heating device, the power supply module is configured for supplying an alternating current to the electromagnetic inductive coil.

Additional aspects and advantages of the present disclosure will be: the present disclosure relates to a heating device and a smoking set having same. As used herein, the heating device includes a heating compartment; an electromagnetic inductive coil wound around a periphery of the heating compartment; the electromagnetic inductive coil at least comprises a first inductive coil and a second inductive coil; the first inductive coil and the second inductive coil are wound around two parts of the periphery of the heating compartment, extending in an axial direction of the heating compartment; turns of the first inductive coil is more than turns of the second inductive coil such that heating speeds of two parts of the periphery of the heating compartment are different. The structure of the heating device and the smoking set as described supra are capable of sectional heating with a simple structure, which may make the smelling and flavoring consistent during smoking.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a cross-sectional view of a low-temperature baked smoking set in accordance with one embodiment of the present disclosure;
FIG. 2 is a partial cross-sectional view of the low-temperature based smoking set in FIG. 1.
FIG. 3 is an isometric view of a wire-wound supporter in the heating device in FIG. 2.
FIG. 4 is an isometric view of the heating tube in accordance with another embodiment of the present disclosure;
FIG. 5 is a cross-sectional of the heating compartment in accordance with another embodiment of the present disclosure;
FIG. 6 is an isometric view of a heating plug-in component in FIG. 5;
FIG. 7 is an isometric view of a paramagnet in accordance with another embodiment of the present disclosure;
FIG. 8a illustrates a magnetic field produced by the electromagnetic inductive coil without the paramagnet in accordance with the present disclosure;
FIG. 8b illustrates a magnetic field produced by the electromagnetic inductive coil with the paramagnet in accordance with the present disclosure;
FIG. 9 is an isometric view of a heating device in accordance with another embodiment of the present disclosure;
FIG. 10 is a block diagram of the power supply module in accordance with the present disclosure.

### DETAILED DESCRIPTION

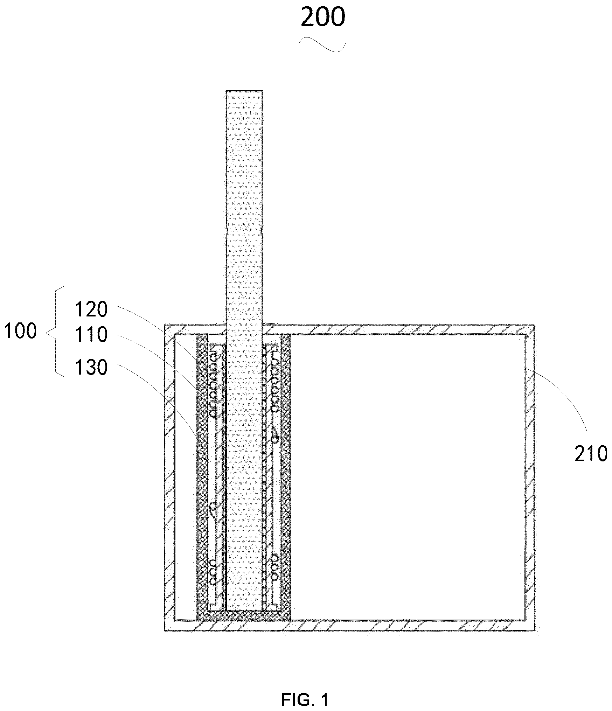

The structure and operating principle of the above heating element with temperature control and the low-temperature baked smoking set having same are illustrated below, mainly shown from FIG. 1 to FIG. 10 in further detail using exemplary embodiments.

Referring to FIG. 1, it is a cross-sectional view of a low-temperature baked smoking set in accordance with one embodiment of the present disclosure. The smoking set 200 includes a heating device 100 and a shell 210; the heating device 100 is disposed inside the shell 210.

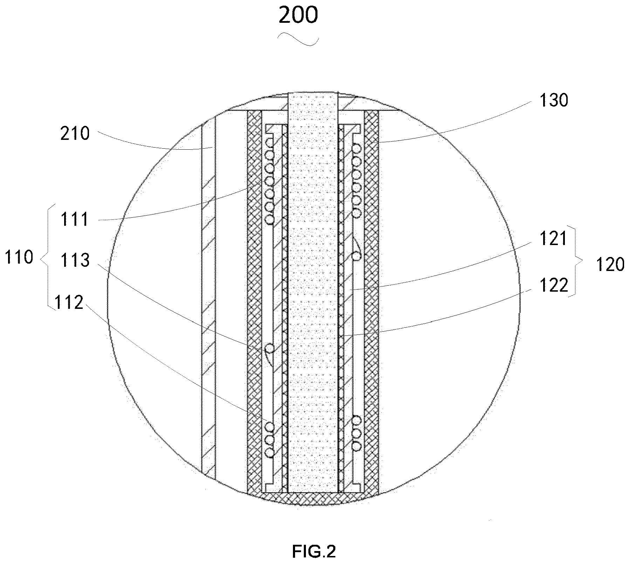

More specifically, with reference to FIG. 2, the above heating device 100 includes an electromagnetic inductive coil 110, a heating compartment 120 and a paramagnet 130; as used herein, the electromagnetic inductive coil 110 wound around a periphery of the heating compartment 120, and the paramagnet 130 is disposed outside the heating compartment 120 and the electromagnetic inductive coil 110, extending along a axial direction of the spiral electromagnetic inductive coil 110.

The above electromagnetic inductive coil 110 may be made of electricity-conductive materials, with a relatively good electricity-conductivity and cheap price such as copper wires and aluminum wires etc. The electromagnetic inductive coil 110 is wound around a periphery of the heating compartment 120 along a certain direction, configured for heating the heating compartment 120. According to law of electromagnetic induction, when the electromagnetic induction coil 110 is supplied with an alternating current, inside of the electromagnetic induction coil 110 generates an alternating magnetic flux. The alternating magnetic flux generates an induced electromotive force in the heating compartment 120, and when the magnetic field lines in the electromagnetic field are passing through the heating compartment 120, the magnetic field lines are cut by the heating compartment 120, the heating compartment 120 generates an inductive current, that is, the vertex current to heat the heating compartment 120.

As used herein, as shown in FIG. 2, the electromagnetic inductive coil 110 includes a first inductive coil 111, a second inductive coil 112 and a connecting coil 113; the first inductive coil 111 and the second inductive coil 112 are wound around two parts of a periphery of the heating compartment 120, the first inductive coil 111 and the second inductive coil 112 extending along an axial direction of the heating compartment 120. Preferably, the first inductive coil 111 is disposed at an end of the heating compartment 120; the second inductive coil 112 is disposed at the other end of the heating compartment 120; turns of the first inductive coil 111 is more than turns of the second inductive coil 112 such that heating speeds at two ends of the heating compartment 120 are different. The connecting coil 113 respectively is connected with the first inductive coil 111 and the second inductive coil 112, and the connecting coil 113 is wound around the middle of the heating compartment 120. In the embodiment, under same circumstances, when turns of the electromagnetic inductive coil is more, the electromagnetic field lines would be more cut by the heating compartment 120 such that the inductive electricity is larger, at last the heating speed is faster. Optionally, when putting the tobacco cigarette into the heating compartment 120, assuming the tobacco cigarette includes a first end, a middle part and a second end, as used herein, the first end is near to the mouthpiece, the second end is always from the mouthpiece, the middle part is between the first end and the second end. The first inductive coil 111 is sleeved on the first end, the second inductive coil 112 is sleeved on the second end, so that the first end has a faster heating speed than the second end of the heating compartment, while the middle part is heated by thermal conductivity from the first end and the second end. The heating speed is changed successively along the first end, the middle part to the second end, from fast to slow. When the first end of the tobacco cigarette is finished to heat, the middle part and the second end still continue heating, then the middle part thereof is finished to heat but the second end still continues heating, which may make the smelling and flavors consistent all the time, without diminished during smoking, therefore improving the user experience.

Understandable, in some embodiments, the connecting coil 113 may be omitted, the first inductive coil 111 and the second inductive coil 112 can be made of one same electricity conductive wire.

Understandable, in some embodiments, outside the electromagnetic inductive coil 110 may be coated with an insulating layer (not shown), the insulating layer is configured to avoid electricity leakage through the electromagnetic inductive layer 110. The insulating layer is made of insulating materials, such as synthetic resins, epoxy resins, phenolic resins, 4250 plastic, polyimide plastic and energy-concentrated plastic etc.

The above heating compartment 120 includes a wire-wound supporter 121 and a heating tube 122. The wire-wound supporter 121 is sleeved on the heating tube 122, the electromagnetic inductive coil 110 is wound around the wire-wound supporter 121, the paramagnet 130 is disposed on the wire-wound supporter 121, the paramagnet 130 is configured to change the magnetic field lines of the electromagnetic inductive coil 110.

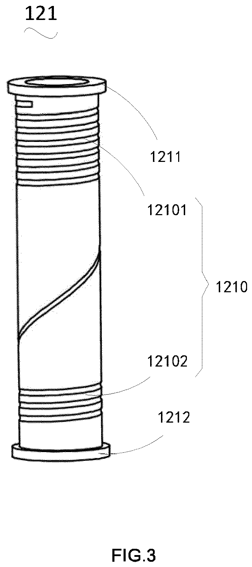

As used herein, with reference to FIG. 3, the wire-wound supporter 121 is made of non-metallic materials or other materials failing to generate electromagnetic induction. The wire-wound supporter 121 has a slot 1210; the slot 1210 includes a first slot 12101 and a second slot 12102. Part of the first induction coil 111 inserts into the first slot 12101, part of the second induction coil 112 inserts into the second slot 12102, around which the electromagnetic induction coil 110 is wound. Two ends of the wire-wound supporter 121 respectively have a first retaining part 1211 and a second retaining part 1212, as used herein, the first retaining part 1211 and the second retaining part 1212 are shaped as a circular ring, the first retaining part 1211 and the second retaining part 1212 are all wound around the supporter 121 such that the electromagnetic inductive coil 110 is more stably wound around the supporter 121.

Understandable, in some embodiments, the slot 1210 may be omitted; the electromagnetic inductive coil 110 may be wound around the supporter 121 in other ways, such as coating high temperature resistant glue etc.

The above heating tube 122 is a hollow cylinder, made of metallic conductor, preferably permeability magnetic materials, with consequently better effect of induction heating, such as powdered iron core, Fe-Ni 50 alloys, Fe-Si-AI composition and Fe-Ni-Mo composition etc. The heating tube 122 has a glossy surface, leading to a better effect of electromagnetic induction heating. The heating tube 122 has a receiving chamber (not shown) therein, the receiving chamber is configured for receiving the aerosol-formed materials, as used herein, the aerosol-formed materials may be at least one or more tobacco bar or flake selected from a group of tobacco shreds, tobacco sheets and tobacco powders.

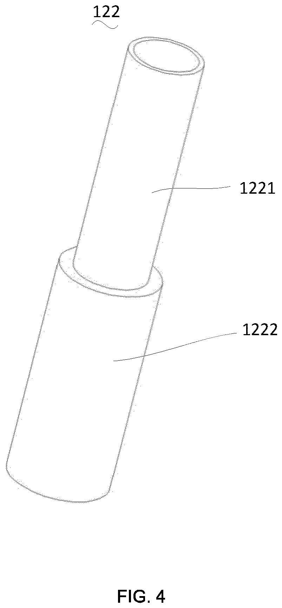

Understandable, in some embodiments, as shown in FIG. 4, the heating tube 122 may include a first heating tube 1221 and a second heating tube 1222, the first heating tube 1221 is connected with the second heating tube 1222, the receiving chamber is communicated with the first heating tube 1221 and the second heating tube 1222, meanwhile, a central axis of the first heating tube 1221 is aligned with the central axis of the second heating tube 1222. An inner diameter of the first heating tube 1221 is equal to that of the second heating tube 1222. An outer diameter of the first heating tube 1221 is less than that of the second heating tube 1222 so that a mass of the first heating tube 1221 is greater than that of the first heating tube 1221. The area of winding the first inductive coil 111 is corresponding to the first heating tube 1221 while the area of the winding the second inductive coil 112 is corresponding to the second heating tube 1222, under same circumstances, for rising to same temperature, the greater the mass of a body, the greater is time needed, therefore, the heating speed of the first heating tube 1221 is greater than the second heating tube 1222.

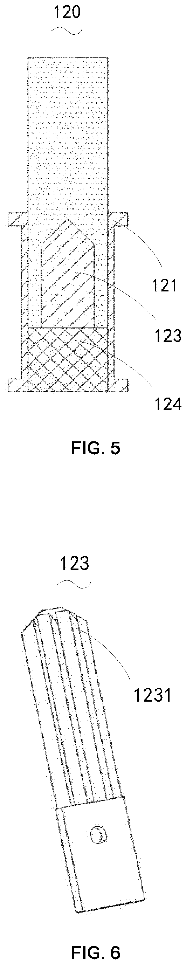

Understandable, in some embodiments, as shown in FIG. 5 and FIG. 6, the heating tube 122 may be omitted, the above heating compartment 120 includes a wire-wound supporter 121, a heating plug-in component 123 and a base 124. The heating plug-in component 123 is configured for being inserted into the aerosol-formed materials; the heating plug-in component 123 is disposed on the base 124. The wire-wound supporter 121 is sleeved on the heating plug-in component 123 and the base 124. The electromagnetic inductive coil 110 is wound around the supporter 121. The paramagnet 130 is disposed on the supporter 121. In this case, the supporter 121 is the same as described supra. The heating plug-in component 123 roughly is shaped as plate-shaped component, made of metallic materials, like pure metals, alloys, metallic compounds and special metallic materials etc. such as Fe, Cu, Al, Sn, Ni, Au, Ag, Pb, Zn and their alloys etc. The heating plug-in component 123 has fins 1231. The shape of the fin 1231 may be designed as a prism in favor of the heating plug-in component 123 to be inserted into the solid aerosol-formed materials, and enlarging the contact area for heating. The base 124 is fixed with the supporter 121 to support the heating plug-in component 123 to make the heating plug-in component 123 available for vertical setting. By replying on the heating plug-in component 123 and the base 124, they make the heating device 100 available for central heating, to satisfy multiple kinds of heating methods.

The above paramagnet 130 is made of at least one or more selected from a group of manganese ferrite, zinc ferrite, nickel-zinc ferrite and manganese-magnesium-zinc ferrite. The paramagnet 130 is barrel-shaped dowel joint element with an end opened. The paramagnet 130 is sleeved on the heating compartment 120. The paramagnet 130 and the heating compartment 120 clamp the electromagnetic inductive coil 110 therebetween.

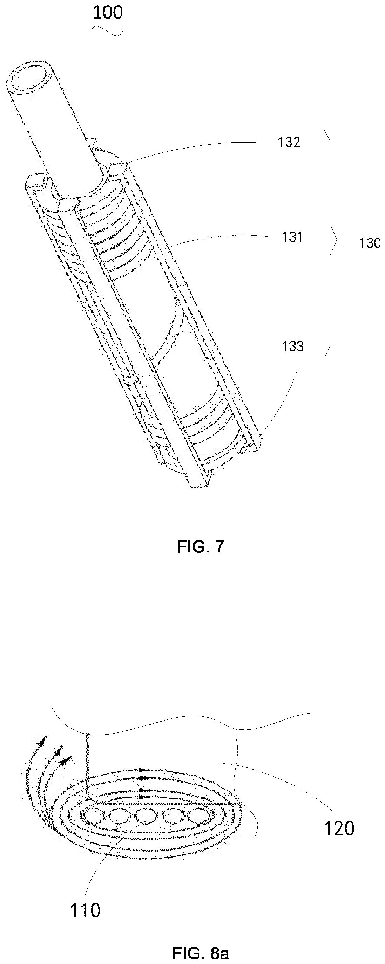

Understandable, in some embodiments, as shown in FIG. 7, the above paramagnet 130 includes a pressing part 131, a first fixing part 132 and a second fixing part 133; the first fixing part 132 and the second fixing part 133 are respectively disposed at two ends of the pressing part 131; the first fixing part 132 is fixed with one end of the heating compartment 120, the second fixing part 133 is fixed with the other end of the heating compartment 120; the pressing part 131 and the heating compartment 120 clamp the electromagnetic inductive coil 110 therebetween. As used herein, the first fixing part 132 may be fixed with the first retaining part 1211 of the supporter 121, the second fixing part 133 may be fixed with the second retaining part 1212 of the supporter 121. The paramagnets 130 may have at least one paramagnet 130 that are evenly disposed around the heating compartment 120's periphery. Optionally, the number of the paramagnets 130 may be four and four paramagnets are evenly disposed around the heating compartment 120's periphery. By contrary, the setting as described supra may improve the efficiency of electromagnetic inductive heating as well as decreasing the materials

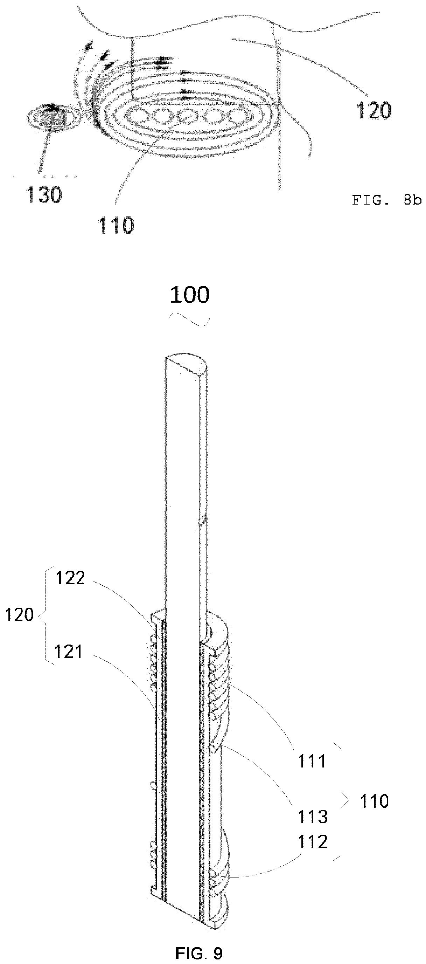

In the embodiment, as shown in FIG. 8a, without the paramagnet 130, the electromagnetic inductive coil 110 is supplied with alternative current to produce the magnetic field, part of the magnetic field lines in the magnetic field are passing through the heating compartment 120, while cut by the heating compartment 120, thus an inductive current in the heating compartment 120 is generated so as to heat the heating compartment 120. The other part of the magnetic field lines in the magnetic field fail to be cut by the heating compartment 120, so that the magnetic field produced by the electromagnetic inductive coil 110 won't entirely be used to generate the inductive current, wasting a lot of time. As shown in FIG. 8b, when the paramagnet 130 is sleeved outside the heating compartment 120, the electromagnetic inductive coil is supplied with the alternative current, to generate the magnetic field. Part of the magnetic field lines in the magnetic field is passing through the heating compartment 120, the other part of the magnetic field lines in the magnetic field are not supposed to pass through the heating compartment 120 but since affected by the paramagnet 130, the direction of the magnetic field lines are changed, the other part of the magnetic field lines passes through the heating compartment 120. Therefore, the inductive current generated by the heating compartment 120 gets larger, with consequently improving the efficiency of electromagnetic inductive heating.

The heating device 100 in accordance with the embodiment includes the electromagnetic inductive coil 110 and the heating compartment 120. By arranging different turns of the electromagnetic inductive coils 111 at two ends of the heating compartment 120, the heating speeds of two ends of the heating compartment 120 are different, so that the heating device 100 in the embodiment has simple structure and is available for sectional heating, so the smelling during smoking keep consistent. Moreover, the paramagnet 130 is sleeved on the heating compartment 120 so that the direction of electromagnetic field lines of the electromagnetic inductive coil 110 is changed, thus improving efficiency of heating by electromagnetic induction.

Understandable, in some embodiments, as shown in FIG. 9, the paramagnet 130 can be omitted. The heating device 100 includes the electromagnetic inductive coil 110 and the heating compartment 120. As used herein, the electromagnetic inductive coil 110 and the heating compartment 120 are the same as described supra. However, the electromagnetic inductive coils 110 at two ends of the heating compartment 120 are set to owe different turns, in this way, the heating speeds at two ends of the heating compartment 120 are different to make the heating device 100 available of sectional heating, therefore the smelling and flavors during smoking keep consistent.

The above shell 210 may be a plastic shell, preferably some plastic shells with good thermal insulation and heat preservation, such as polycarbonate, polyurethane and polyimide etc. The shell 210 is configured for receiving the heating device 100.

In some embodiments, the shell 210 is made by a metallic shell, covering with a plastic membrane to contribute to heat insulation and thermal preservation.

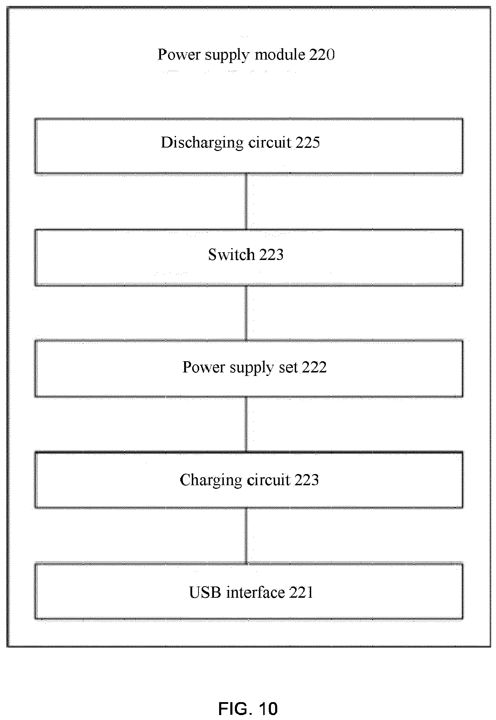

As used herein, the above smoking set 200 further includes a power supply module 220, the power supply module 220 is coupled with the electromagnetic inductive coil 110 to supply an alternative current for the electromagnetic inductive coil 110, so the electromagnetic inductive coil 110 is capable of heating. Optionally, as shown in FIG. 10, the power supply module 220 includes an USB interface 221, a power supply set 222, a switch 223, a charging circuit 224 and a discharging circuit 225. The power supply 222 is coupled with the input end through the switch 223; the output end of the discharging circuit 225 is coupled with the electromagnetic inductive coil 110, the power supply set 222 is coupled with the USB interface 221 through the charging circuit 224. More specifically, the power supply set 222 may be a charging power supply set 222. The switching 223 is disposed on a surface of the shell 210. The product will be interpreted hereinafter in four cases, the first case: when the user turns off the switch 223, the power supply set 222 is coupled with the input end of the discharging circuit 225, the discharging circuit 225 will be in the discharging work state to supply power to the electromagnetic inductive coil 110 via the output end itself, so that the electromagnetic inductive coil 110 generates an excitation field; the excitation field causes the heating tube 122 in the heating compartment 120 or the heating plug-in component 123 to generate heating itself, so as to heating the aerosol-formed materials, as a result, the user may smoke the smoking set 100 after closing the switch 223. The second case; when the user turns off the switch 223, the power supply set 222 is unconnected with the input end of the discharging circuit 225. When the discharging circuit 225 is not in working state, the user fails to smoke the smoking smog generated by the smoking set 100. The third case: when the USB interface 221 is supplied with external power supply, the charging circuit 224 detects the existing of the external power supply and comes into the electricity charging state, so as to charge the chargeable power supply. The fourth case: when the USB interface 221 isn't connected with the external power supply, the charging circuit 224 doesn't detect the existing of external power supply, consequently the charging circuit 224 is in non-working state. Optionally, the power supply set 22 includes a chargeable lithium battery.

The smoking set 100 in accordance with the present disclosure, includes a heating device 100 for improving the heating efficiency of the electromagnetic induction, effectively improving the user experience, and effectively improving the heating efficiency, as well as improving the utilization rate of energy, with a simple structure and low price.

Terminology used herein is for the purpose of describing particular embodiments only and is not intended to be limiting of the invention. Variations may be made to the embodiments and methods without departing from the spirit of the disclosure. Accordingly, it is appropriate that the appended claims be construed broadly and in a manner consistent with the scope of the disclosure.

## Claims

1. A heating device comprises:
a heating compartment; and
an electromagnetic inductive coil, wound around a periphery of the heating compartment;
**characterized in that**, the electromagnetic inductive coil at least comprises a first inductive coil and a second inductive coil; the first inductive coil and the second inductive coil are wound around two parts of the periphery of the heating compartment, the first inductive coil and the second inductive coil extending in an axial direction of the heating compartment;
turns of the first inductive coil is more than turns of the second inductive coil such that heating speeds of two parts of the periphery of the heating compartment are different.

2. The heating device of claim 1, **characterized in that**, the first inductive coil and the second inductive coil are disposed at two ends of the heating compartment.

3. The heating device of claim 1 or 2, **characterized in that**, the electromagnetic inductive coil further comprises a connective coil, respectively connected with the first inductive coil and the second inductive coil.

4. The heating device of claim 3, **characterized in that**, the first inductive coil and the second inductive coil are made by one same electricity-conductive wire.

5. The heating device of claim 1 or 2, **characterized in that**, the heating device further comprises a paramagnet, disposed outside the heating compartment and the electromagnetic inductive coil, extending along an axial direction of the spiral electromagnetic inductive coil; the paramagnet is configured for changing a direction of magnetic field lines of the electromagnetic inductive coil.

6. The heating device of claim 5, **characterized in that**, the heating compartment further comprises a metallic heating tube and a wire-wound supporter; the heating tube is configured for receiving aerosol-formed materials; the wire-wound supporter is sleeved outside the heating tube, the electromagnetic inductive coil is wound around the supporter; the paramagnet is disposed outside the supporter.

7. The heating device of claim 5, **characterized in that**, the heating compartment comprises a wire-wound supporter, a metallic heating tube and a base, the heating tube is configured for receiving aerosol-formed materials; the supporter is sleeved outside the heating tube and the base, the electromagnetic inductive coil is wound around the supporter; the paramagnet is disposed outside the supporter.

8. The heating device of claim 6 or claim 7, **characterized in that**, the supporter comprises a slot, the slot comprises a first slot and a second slot, part of the first inductive coil is inserted into the first slot and part of the second inductive coil is inserted into the second slot.

9. The heating device of claim 6 or claim 7, **characterized in that**, the paramagnet comprises a pressing part, a first fixing part and a second fixing part; the first fixing part and the second fixing part are respectively disposed at two ends of the pressing part; the first fixing part is fixed with one end of the heating compartment, the second fixing part is fixed with the other end of the heating compartment; the pressing part and the heating compartment clamp the electromagnetic inductive coil therebetween.

10. The heating device of claim 9, **characterized in that**, a number of the paramagnets is multiple, and multiple paramagnets are evenly distributed around the heating compartment.

11. The heating device of claim 6 or claim 7, **characterized in that**, the paramagnet is hollow and a tubular element with an open end, the paramagnet is sleeved on the heating compartment, the paramagnet and the heating compartment clamp the electromagnetic inductive coil therebetween.

12. The heating device of any one of claims 5-11, **characterized in that**, the paramagnet is made of at least one or more selected from a group of manganese ferrite, zinc ferrite, nickel-zinc ferrite and manganese-magnesium-zinc ferrite.

13. A smoking set comprising:
a heating device of any one of claims 1-12; and
a power supply module, the power supply module is coupled with the electromagnetic inductive coil in the heating device, the power supply module is configured for supplying alternating current to the electromagnetic inductive coil.
